# EUROPEAN PATENT SPECIFICATION

(11) **EP 2 150 272 B1**
(45) Date of publication and mention of the grant of the patent: **14.08.2013**
(21) Application number: 08763540.5
(22) Date of filing: 10.06.2008
(51) Int. Cl.: A61K 38/28, A23K 1/18, A23K 1/165, A61P 1/14

(54) **MEANS AND METHODS FOR ENHANCING WEIGHT GAIN IN POULTRY**
MITTEL UND VERFAHREN ZUR ERHÖHUNG DER GEWICHTSZUNAHME BEI GEFLÜGEL
MOYENS ET PROCÉDÉS POUR AMÉLIORER UN GAIN DE POIDS CHEZ DES VOLAILLES

(30) Priority: 07.06.2007 US 925000 P
(43) Date of publication of application: 10.02.2010
(73) Proprietor: Nutrinia Ltd., 17000 Nazareth Illit (IL)
(72) Inventor: SHEHADEH, Naim, 24908 Kfar-Yasif (IL); ESHKAR-SEBBAN, Lora, 44857 Beit Hashmonai (IL); HANIEN, Aviv, 34750 Haifa (IL); DEVIR, Sharon, 40200 Kfar Vitkin (IL)
(74) Representative: Straus, Alexander
(86) International application number: PCT/IL2008/000782
(87) International publication number: WO 2008/149374

(56) References cited:
- EP-A- 0 315 471
- WO-A-2005/115473
- WO-A-2005/117951
- WO-A2-2004/112494
- US-A- 5 985 336
- SHEHADEH N ET AL: "INFLUENCE OF ORAL INSULIN SUPPLEMENTATION ON CARBOHYDRATE, LIPID AND PROTEIN METABOLISM IN WEANED BALB/C MICE" JOURNAL OF PEDIATRIC ENDOCRINOLOGY AND METABOLISM, FREUND PUBLISHING HOUSE, TEL AVIV, vol. 16, no. 3, 1 January 2003 (2003-01-01), pages 431-437, XP009054182 ISSN: 0334-018X
- SHULMAN R J: "ORAL INSULIN INCREASES SMALL INTESTINAL MASS AND DISACCHARIDASE ACTIVITY IN THE NEWBORN MINIATURE PIG" PEDIATRIC RESEARCH, vol. 28, no. 2, 1990, pages 171-175, XP009107091 ISSN: 0031-3998

## Description

### FIELD OF THE INVENTION

The present invention relates to means and methods for enhancing intestinal function in poultry, leading to an increase in food conversion ratio and in total weight gain. Particularly, the present invention relates to insulin-containing feed formulations enhancing intestinal function and weight gain in poultry.

### BACKGROUND OF THE INVENTION

Productivity of a poultry flock largely depends on the food conversion ratio (FCR), i.e. the amount of fodder required to achieve a certain amount of product (poultry meat). It has been previously shown that improving the nutrition quality of the feed increased poultry growth rate in modem strains. The increased growth is reflected in either an increased weight of the adult chicken, or a reduction in the period of time required for obtaining an adult chicken.

Materials promoting growth, the so-called "growth stimulants", are typically employed in animal feed for producing quicker growth and increased meat tissue production. Known growth promoting materials may be categorized as antibiotics, synthetic chemical growth promoters, or hormones, particularly sexual hormones.

For example, U.S. patent No. 4,929,600 discloses a method for improving the carcass quality of poultry, via manipulation of the hormone system of the poultry including altering blood levels of hormones in the bodies of poultry by adding effective amounts of metabolically-active thyroid hormone.

U.S. Patent No. 5,017,560 discloses the administration of porcine prolactin (pPRL) to poultry in dosages of from about 1-500 µg/kg/day, preferably 10-100 µg/kg/day, to promote growth by improving the rate of weight gain and/or increasing feed utilization efficiency.

Mineral feed supplemen ts for poultry has also been disclosed as a means to improve weight gain. U.S. Patent No. 5,459, 162 discloses a copper feed supplement for chickens and method of using same. U.S. Patent No. 7,045,150 discloses a nutrient formulation containing tellurium for use in poultry, and a method of feeding it which improves subsequent viability, cumulative feed efficacy or weight gain.

U.S. Patent No. 5,985,336 discloses nutrient formulation including moisture which is designed for use in poultry and other animals, and a method of feeding it which improves subsequent viability, cumulative feed efficiency and weight gain. The method comprises making available for consumption *ad libitum* a high moisture material containing at least about 20% by weight water to the poultry or other animals before they are offered dry food *ad libitum.*

U.S. Patent No. 6,258,399 discloses a composition containing monosaccharides, disaccharides, oligosaccharides fed immediately after hatching and during the first days of life that has a growth enhancing and mortality reducing effect. International Application Publication No. WO 2007/015932 relates to supplementing a poultry diet with phosphorylated glucomannan polysaccharides to reach increased rate of poultry weight gain, more efficient feed-to-gain and increased size of the poultry breast meat.

U.S. Patent application Publication No. 20030099624 discloses the administration of spores or live cells of *Bacillus laterosporus* to poultry, which cause one or more of: (i) weight gain; (ii) increased feed conversion to mass; and (iii) decreased mortality. International Application Publication No. WO 01/87085 relates to animal feed, particularly poultry or pig fed suitable for feeding at the animal's early life stage including a fungi chosen from a genera within the Basidiomycetes, an extract, a derivative or a mixture thereof, and use thereof for the improvement of weight gain and/or for reducing the feed conversion and/or for the improvement of the feed value and/or health and well-being of the animal.

Bioactive compounds naturally present in unprocessed milk and eggs have been shown to have a positive effect on developmental, immunological and nutritional aspects in several human and commercially viable livestock. Among them insulin has been suggested as one of the trophic factors present in colostrum. The concentration of insulin in human and pig colostrum is 3-30 fold greater than that in the serum.

Insulin has been shown both *in vitro* and *in vivo* to accelerate a number of gastrointestinal (GI) functions. Oral intake of insulin by newborn pigs and rats has been shown to enhance development of intestinal activity and growth. Enteral administration of insulin was found to be beneficial in reducing food intolerance in human preterm infant (Shulman R J 1990. Pediatr Res 28:171-175; Shulman R J 2002. Arch Dis Child Fetal Neonatal Ed 86:F131-F133).

International (PCT) Patent Application Publication No. WO 2005/115473 to some of the inventors of the present invention and co-workers showed that insulin and insulin derivatives provided to chicks through drinking water or feed resulted in a higher weight gain of the chicks compared to those received same feed without insulin. However, to achieve these results high amounts of insulin and at least 21 days of insulin administration were required.

WO 2005/117951 refers to a method for treating short bowel syndrome by increasing intestinal function by oral and/or enteral administration of a therapeutically effective amount of insulin to a subject in need thereof. The amount is indicated to range from 1 µIU to 10,000 IU. In the examples provided the pharmaceutical composition comprising insulin is administered to rats and humans, respectively.

Shehadeh et al., Journal of Pediatric Endocrinology and Metabolism, Tel Aviv, 16(3) 203, pp. 431-437 describes the local and systemic effects of oral insulin supplementation in the post weaning period, using mice as a model.

WO 2004/112494 refers to an infant feed supplement comprising an encapsulated bioactive molecule such as insulin.

In recent years, the public awareness regarding the safety of food products, particularly fresh produce has been raised significantly. There is a growing demand for poultry and other meat products which are free of growth promoters, particularly antibiotics and hormones. However, as described hereinabove, growth stimulants increase the food conversion ratio and the economical value of a poultry flock.

Thus, there is a recognized need for and it would be highly advantageous to have bioactive agents that act as a poultry growth promoter while being added at low concentrations and for a short duration, such that no traces of this agent may be found in the final meat product.

### SUMMARY OF THE INVENTION

The present invention successfully addresses the shortcomings of the known compositions and methods by providing specific treatment regimens for increasing weight gain in poultry and improving intestinal function as well as enhancing the health, viability and feed efficiency in poultry.

The present invention relates the use of to insulin-containing compositions effective in increasing poultry intestinal activity, particularly increasing the intestinal mass, and in increasing the weight gain of chicks without negatively affecting the meat quality.

The present invention is based in part on the unexpected discovery that administering mammalian insulin, particularly recombinant human insulin to chicks shortly after hatching, even when utilizing reduced dosage and application times achieves significant growth promotion.

Without wishing to be bound by any particular theory or mechanism of action the poultry growth promotion may be attributed to the direct activity of insulin on the chick intestine, resulting in increased intestinal mass and activity, leading to an increase in feed conversion ratio and weight gain.

Moreover, the teachings of the present invention are advantageous over previously known methods for increasing weight gain in commercially grown poultry flocks in that (i) the amount of insulin required to achieve significant weight gain is significantly lower compared to hitherto known compositions and methods; (ii) the insulin administration period is short, and thus (iii) marketed poultry meat is free of growth promoting agents.

A method may be provided for enhancing at least one outcome selected from improved health, viability, cumulative weight gain and feed conversion efficiency of poultry, comprising orally administering insulin at a cumulative dose of 2 IU or less per individual chick.

A plurality of these beneficial outcomes may be achieved.

Methods for enhancing a plurality of outcomes selected from improved health, viability, cumulative weight gain, or feed conversion efficiency of poultry using insulin amounts at doses at least one order of magnitude lower compared to hitherto used doses may be provided. The present invention now discloses that the effect of various insulin amounts on poultry intestinal mass and weight gain shows an optimal curve, utilizing lower doses of insulin than hitherto thought efficient, with higher insulin amounts having no or negative effect on these parameters.

The amount of insulin may be effective in increasing the poultry intestinal mass. The amount of insulin is less than I IU per chick or less. According to certain preferred embodiments, the cumulative amount of insulin is 0.5 IU per chick or less, typically in the range of from about 0.004 IU to about 0.5 IU cumulative insulin amount per chick.

The insulin may be mammalian insulin. The insulin may be recombinant or semisynthetic human insulin.

The present invention shows that insulin administration can start shortly after hatching, such that it may be easily administered orally with no need to in-ovo injection of the insulin to the pre-hatched avian chick.

According to one aspect the present invention provides the use of a feed supplement comprising insulin for at least one of improved cumulative weight gain and improved feed conversion efficiency of poultry, wherein the feed supplement is administered at a total cumulative dose of insulin of 1 IU or less per individual chick.

According to an embodiment the feed supplement is used for increasing the intestinal mass of the poultry compared to the intestinal mass of poultry not receiving the feed supplement.

According to another embodiment, the feed supplement is administered at a total cumulative dose of insulin of about 0.5IU per chick or less.

According to still another embodiment, the feed supplement is administered at a total cumulative dose of insulin of from about 0.004 IU to about 0.5 IU per chick.

Administration of insulin may start shortly after hatching, one day, two days or three days after hatching. insulin administration may start when the poultry are one day old.

Insulin can be administered directly or as an oral feed supplement. The insulin or insulin comprising composition can be mixed with animal feed or drinking liquid (e.g. water). The insulin may be administered as a feed supplement, premix, drinking water supplement, bolus and/or microcapsule. Insulin may be encapsulated in an encapsulating material. Encapsulating materials are typically selected from the group consisting of polysaccharide, maltodextrin, milk powder, whey protein, lipid, gum Arabic and microcrystalline cellulose. Other encapsulation materials are well known in the art

Insulin may be microencapsulated within a matrix of maltodextrin (MD) and vitamin C to form feed supplement. This formulation is highly stable, particularly to heat. The feed supplement may be further mixed with a feed formula.

It is shown that oral administration of insulin at a total amount of 2 IU per chick or less, for a period as short as about 7 days starting at the first day after hatching, increases the cumulative weight gain significantly. Even when insulin is administered only during the first week of the chick's life, increase in weight gain is pronounced up to the poultry marketing date, typically at age of 35-42 days. This finding is highly advantageous in the poultry industry, answering the growing demand for fresh meat with no traces of growth promoting agents.

Insulin may be administered from about the first day after hatching until about 7 days after hatching, Insulin administration may be stopped at least 7 days before marketing, or 14 days, or 21 days, or 28 days before marketing.

The use of insulin for the preparation of a poultry feed supplement for enhancing at least one of, or according to certain embodiments a plurality of outcomes selected from improved health, viability, cumulative weight gain and feed conversion efficiency in poultry is disclosed, wherein the feed supplement may be administered at a total does of insulin of 2 IU or less per individual chick.

According to certain embodiments, the feed supplement is administered at a total dose of insulin of about 0.5 IU per chick or less. According to other embodiments, the feed supplement is administered at a cumulative dose of insulin of from about 0.004 IU to about 0.5 IU per chick.

The feed supplement administration may start between immediately after hatching to about three days after hatching, typically when the poultry are one day old. The feed supplement may be administered for at least 7 consecutive days. The feed supplement administration may be stopped at least 7 days, at least 14 days, at least 21 days or at least 28 days before the scheduled marketing day.

The insulin in the poultry feed supplement may be encapsulated within encapsulating material, typically selected from the group consisting of polysaccharide, maltodextrin, milk powder, whey protein, lipid, gum Arabic, vitamins or microcrystalline cellulose. The encapsulating material may comprise maltodextrin and vitamin C.

Typically, the feed supplement may be provided to the poultry mixed with its regular feed formula or drinking water.

An avian feed supplement composition may be provided, comprising insulin encapsulated in an encapsulating material, wherein the insulin amount is from about 0.5 IU to about 5 IU per gram dry composition. The feed supplement composition may be in a form of a dry powder. The encapsulating material is typically selected from the group consisting of polysaccharide, maltodextrin, milk powder, whey protein, lipid, gum Arabic or and microcrystalline cellulose. The encapsulating material may be a combination of maltodextrin and vitamin C, and the feed supplement composition is prepared as described hereinbelow.

The insulin amount in the feed supplement is from about I IU to about 3 IU. The insulin amount may be from about 1 IU to about 2 IU.

An avian feed supplement composition may be provided comprising insulin for enhancing at least one outcome selected from improved health, viability, cumulative weight gain and feed conversion efficiency of poultry, wherein the composition is administered at a dose of insulin of about 2 IU or less per individual chick.

Other objects, features and advantages of the present invention will become clear from the following description and drawings.

### DETAILED DESCRIPTION OF THE INVENTION

The present invention relates to means and methods of increasing at least one effect, preferably a plurality of effects selected from increased body weight, and/or intestinal mass and/or function, and/or health, and/or viability and/ or feed efficiency in immature members of avian species, e.g., in poultry. The present invention now discloses that oral administration of insulin from about the first day to about the seventh day after hatching at a cumulative amount of 2 IU or less insulin per individual chick results in significant weight gain at the end of the poultry growth period.

Before explaining at least one embodiment of the invention in detail, it is to be understood that the invention is not limited in its application to the details set forth in the following description or exemplified by the examples. The invention is capable of other embodiments or of being practiced or carried out in various ways. Also, it is to be understood that the phraseology and terminology employed herein is for the purpose of description and should not be regarded as limiting.

### Definitions

As used herein the term "poultry" refers to domesticated fowl kept primarily for meat and eggs; including birds of the order Galliformes, e.g., the chicken, turkey, guinea fowl, pheasant, quail, and peacock; and Anserigormes (swimming birds,) e.g., the duck and goose. The term also refers to pigeons, doves, or game birds like pheasants. Accordingly, the term "chick" refers to the young of the above-described birds.

As used herein, the term "insulin" refers to a polypeptide hormone, which is naturally secreted by the islets of Langerhans and functions in the regulation of the metabolism of carbohydrates and fats, particularly the conversion of glucose to glycogen. According to certain embodiments, the term refers to insulin which can be used to increase the birds' body weight. This may be native insulin (purified or synthetic or recombinant) or analogs thereof. The term further includes functional derivatives of insulin, referring to the products of enzymatic digestion of insulin (e.g. by trypsin, chemotrypsin, lysine-C or elastase). According to certain embodiments, the term insulin refers to mammalian insulin. According to certain typical embodiments, the term insulin refers to recombinant human insulin and to analogs thereof.

As used herein, the term "IU" (International Unit) refers to the biological equivalent of about 45.5 µg pure crystalline insulin (exactly 1/22mg),

As used herein, the term "feed supplement" refers to compositions comprising pharmaceutical or nutritional substances that are not natural feedstuffs, and that are added to made-up and stored feeds for various purposes, mainly to control infectious disease or, as in the present invention, to promote growth.

The terms "increasing" or "enhancing" (e.g. body weight) refers to at least 0.05%, 0.1%, 0.5%, 1%, 2%, 5%, 10%, 15%, 20% increase in an examined parameter of the present invention including body weight, feed efficiency, intestinal function or intestinal mass in a poultry treated with insulin as compared to non-treated poultry.

As used herein the term "about" refers to ±10%. It is required that any numeric value in the application will be referred to as if it is preceded by the term "about".

The present invention may provide a method for enhancing at least one parameter selected from improved or enhanced health, viability, cumulative weight gain and feed conversion efficiency of poultry, comprising orally administering insulin at a total dose of 2 IU or less per individual chick.

Insulin has been suggested as one of the trophic factors present in colostrum of several mammalian species, including human and pigs. Its concentration in human and pig colostrum is 3-30 fold greater than that in serum and decreases in parallel to decrease in the colostrum trophic activity. Shulman (1990, ibid) demonstrated that intestine ileal mass and lactase activity increased in newborn miniature pigs in response to oral administration of insulin. The increase in the ileal mass was significant enough to affect the total small intestinal mass, which was found to be higher in groups treated with insulin compared to non-treated groups. In this and other studies it has been shown that oral intake of insulin in the concentrations examined does not affect the blood sugar or insulin level, indicating that insulin is not absorbed systemically to any significant degree. Shulman (2002, ibid) further showed that enteral administration of insulin to preterm human infants enhances gastrointestinal function, as measured by increased lactase activity.

Insulin was also detected in poultry embryos before β-cells are recognizable, as well as in the egg constituents even before fertilization. However, the concentration of insulin in the yolk and white of unfertilized and fertilized eggs is much lower compared to its concentration in the embryo (0.2-0.8 ng/ml vs. 2 ng/g) (de Pablo F et al., 1982. Endocrinology 111:1909-1916). The site of yolk synthesis is primarily the hen's liver and it is transported to the developing ovum (Knepper P A 1999. In Vitro Cell. Dev. Biol. Animal 35:357-363).

In keeping of animals, the economy of meat production is significantly influenced by the extent of utilization of the fodder used for feeding the animals, i.e. the amount of fodder required to achieve a certain weight gain. Another important parameter is the period during which the animals reach the desired marketing weight. The lower the fodder amount required and the shorter the time of feeding required to achieve the slaughter weight, the more economical is the meat production.

Commercial growth conditions put young poultry under stress, mainly due to high growth densities and long light periods. Such stress cannot be removed; however, efficient growth promoting agents help the chicks to overcome these conditions.

The present inventors and co-workers have previously demonstrated preliminary results showing that oral administration of an amino acids complex comprising degraded insulin to Cobb male chicks for a period of 21 days at concentrations of from above 2 IU to about 4 IU per chick resulted in a higher weight gain compared to non-treated chicks (WO 2005/115473).

It is shown that insulin administration for as short period as about seven days at a cumulative amount of less than 2 IU per chick suffices to exert the beneficial effect of insulin on the young poultry intestine. Insulin administration should start a short time after hatching, from immediately after hatching to 2-3 days post hatching. Insulin administration may start on the first day after hatching. As disclosed in the example section hereinbelow, such insulin administration results in an increase in weight gain of treated animals compared to non-treated animals up to the poultry marketing day.

Shortening the insulin administration time period is of significant importance for the poultry industry. There is a growing awareness of the regulatory authorities as well as the public regarding consumption of food containing potentially health-hazardous compounds. Accordingly, fresh meat which is free of traces of growth promoters has higher economical value. The disclosure provides for a significant weight gain up to the marketing day while insulin administration is ceased well before this day, such that no traces of exogenous insulin may be found in the marketed poultry. Taking together the basic low cost of insulin with the short administration time, low insulin amounts required, significant weight gain and no insulin residues at marketing, use of insulin as a growth promoter according to teachings of the present invention is highly advantageous for the poultry industry.

Surprisingly, the present invention now shows that intact mammalian insulin is highly effective in increasing poultry weight gain. Moreover, the present invention shows for the first time that oral administration of insulin, particularly human insulin, increases the poultry intestinal mass.

Insulin is considered as a highly conserved protein when mammalian proteins are compared. For example, bovine and porcine insulin are very similar to human insulin. In terms of amino acid sequence, seven amino acids differences are detected when avian insulin and human insulin are compared.

Without wishing to be bound by any specific mechanism or theory, the observed increase in intestinal mass may contribute to the increased weight gain by enhancing the feed conversion efficacy or enhancing the amount or rate of food consumption. It is to be noted that the increase in weight gain not only did not affect the general health and viability of the treated animal negatively, but to the contrary, enhanced it. This is reflected by the increase in breast muscle weight in treated poultry and no change in the percentage of fat.

Insulin is administered at a cumulative dose of 1 IU per chick or less. According to certain preferred embodiments, the cumulative amount of insulin is 0.5 IU per chick or less, typically in the range of from about 0.004 IU to about 0.5 IU per chick.

Insulin can be orally administered directly or in an oral composition, alone or with the poultry feed or drinking water.

The insulin may be encapsulated within encapsulating material providing stability to the insulin. As used herein, the term "insulin stability" refers to maintaining at least 80%, 85%, 90% 95% or 100% of the insulin initial activity. Methods of encapsulating insulin are known in the art. Examples of such methods are provided in International Patent Applications Publication Nos. WO 2004/112494 and WO 2005/115473, assigned to the Applicant of the present invention.

In the food and pharmaceutical industries, for example, microencapsulation is used to stabilize the core material, to control the timing and rate of the release of the core material and to separate and prevent chemical interaction between reactive or incompatible components of a multicomponent formulation. Thus, microencapsulation makes it possible to protect sensitive bioactive agents, to ensure against activity loss and to mask or preserve flavors and aromas. Encapsulation may be used to preserve biological activity of bioactive ingredient, such as growth promoting agents against any of the following or similarly destructive factors: adverse temperature, pressure, humidity, pH, osmotic concentration, ionic concentration, chemical degradation, presence of metals, surfactants and chelators, radiation (including but not limited to UV, IR, visible light), enzymatic and microbial degradation and combinations thereof.

Release of the encapsulated bioactive ingredient may occur spontaneously in the digestive tract, or may be the result of environmental events.

A protective layer surrounding or incorporating the insulin may be specifically designed to degrade, or undergo controlled release as a response to exposure to the change in environmental condition. The change in the environmental condition can be time, temperature, moisture content, pressure, or pH, ionic strength, enzymatic activity, or a combination thereof. The insulin may be encapsulated in a material designed to protect it from digestion in the digestive system of the poultry, and to release the insulin only as a response to an increase in pH. The insulin may be further encapsulated with another encapsulating material, designed to protect the core from increased temperature. The skilled artisan in the art, would recognize that the order of environmental triggers releasing the active compound is not rigid and depends on the environmental conditions of manufacturing, environmental conditions of integration into food or feed products, environmental conditions of storage after integration onto food or feed products, desired delivery location within the gastrointestinal system, timing and physiological activity desired.

Any factor that may affect the entrapment of insulin in a biodegradable matrix and thereby affect its initial loading, subsequent release, or a combination thereof, may be utilized. Such factors may comprise *inter-alia*, the initial solvent concentration, its molecular size and polarity, the temperature and pressure under which the solvent is removed, molecular weight number (MWn) average of the biodegradable matrix, and its polydispersity index. When the biodegradable matrix is a polymer, the size and polarity of the insulin, the monomer ratio and distribution along the copolymer's chain, or a combination thereof may be also considered. In addition, D/L ratio within each monomer of a biodegradable polymer will affect release rates. The term D/L ratio refers to the ratio of monomer molecules that affect the direction (D-right, L-left), in which a cross-polarized lens will be rotated when observing a single optically active monomer like lactic acid. Since most mammals have D-specific enzymes, that ratio will affect the digestion rate of the biodegradable biopolymer, affecting its molecular weight and consequently its viscosity, thereby affecting release rate of the entrapped insulin.

Various materials may be used as the encapsulated material as described in WO 2004/112494 and WO 2005/115473 cited above. Insulin may be microencapsulated within a matrix of maltodextrin (MD) and vitamin C as described in the Example section hereinbelow.

The encapsulated insulin may be further mixed with a feed formula or drinking liquid, typically water. The encapsulation protects the insulin in a manner that, when a drinking liquid or solid feed containing the encapsulated insulin in consumed by a young avian, the insulin is protected, at least partially, during its passage through the two stomachs such that sufficient amounts of insulin is still active to exert its growth-promoting activity as described herein.

The particular poultry feeds which are useful in the present invention are not critical. A variety of poultry feeds are available commercially. Typical poultry feeds which can be useful for providing insulin to young poultry according to the teachings of the present invention are disclosed in M. O. North and D. D. Bell, "Commercial Chick Production Manual," Chapman & Hall, 4th Ed., 1990, and may contain mixtures of the following: carbohydrates, such as, barley; buckwheat; cassava; corn, for example, yellow corn, white corn and high-lysine corn; millet (proso); molasses; oats; rice; rye; sorghums, for example, kafir and milo; triticale; and wheat; mill by-products, such as, hominy feed; rice bran; rice hulls; wheat by-products, for example, wheat bran and wheat millings, shorts; fats and oils, such as, hard fats from slaughtered cattle; soft fats, for example, yellow grease; hydrolyzed animal fats; vegetable oils; and polyunsaturated fatty acids in egg yolks; proteins of animal origin, such as, dried blood; dried poultry waste, for example, dried cage layer manure; liver meal; meat by-products, for example, meat scraps and meat and bone meal; milk products, such as, dried skim milk; dried butter milk, and dried whey; poultry by-products, such as, hydrolyzed poultry feather meal; poultry hatchery by-product meal, for example, eggshells, unhatched and infertile eggs, and culled chicks; proteins of fish origin, such as, white fish meal; dark fish meal; and shrimp meal; proteins of vegetable origin, such as, corn gluten, coconut (copra) oil meal; cottonseed meal; guar meal; linseed (flax) oil meal; peanut (groundnut) meal; rapeseed oil meal (canola meal); safflower meal; sesame meal; soybean meal; full-fat soybeans; and sunflower seed meal; green leafy products, such as, alfalfa products, for example, sun-cured alfalfa meal, dehydrated alfalfa meal, and dehydrated alfalfa leaf meal; macrominerals, such as, curacau (island) rock phosphate (CaHPO₄)(CaHPO₄H₂O); dicalcium phosphate (CaHPO₄2H₂O); rock phosphate; steamed bone meal (Ca₃(PO₄)₂); argonite (CaCO₃); limestone (CaCO₃); oyster shell (CaCO₃); gypsum (CaSO₄2H₂O) and salt (NaCl); and vitamins, minerals and trace ingredient, such as, fat-soluble vitamins, for example, vitamins A, D, E, and K; watersoluble vitamins, for example, C (ascorbic acid), thiamin (B₁), riboflavin (B₂), pantothenic acid, niacin, pyridoxine (B₆), choline, biotin, folacin (folic acid), B₁₂ (cobalamin); minerals, such as, calcium; phosphorus; vitamin D; sodium; chlorine; potassium; sulfur; iodine; fluorine; iron; copper; manganese; magnesium; selenium; vanadium; and zinc; amino acids, such as, methionine; cystine; lysine; tryptophan; and arginine; and other feed constituents, such as, antibiotics; arsenicals; xanthophylls; antioxidants; coccidiostat; electrolytes; pellet binders; tranquilizers and other supplements, for example, flavoring agents, enzymes, thyroactive compounds, and drugs. Typically, basal standard commercial diet used in the present invention is primarily composed of corn, wheat, soybean meal, vitamins, antibiotic growth promoter and coccidiostat premix.

Poultry can be fed with the same fodder formula throughout the growth period. However, current growth regimes comprise particular formula for each growth stage, including pre-starter diet typically provided from the first day after hatching until the 7^{th} day; starter - provided until day 17; grower through day 27 and finisher feed until marketing, typically between 35-42 days after hatching.

As is shown in the Example section hereinbelow, although insulin was administered only for the first seven days of the chick life, higher weight gain was observed in the insulin treated chick compared to non-treated animals up to the age of 28 days.

Insulin may be administered from about the first day after hatching until about 7 days after hatching. Insulin administration may be stopped at least 7 days before marketing, or 14 days, or 21 days, or 28 days before marketing.

The objects, advantages, and novel features of the present invention will become apparent to one of ordinary skilled in the art upon examination of the following examples, which are not intended to be limiting.

### EXAMPLES

### Example 1: Treatment of Poultry with Insulin Derivatives

The following experiment is a comparative example representative of the prior art brought for a comparison with the study of the present invention. The experiment was conducted to investigate the performance of broilers given an animal feed additive developed and manufactured by Nutrinia Ltd. (Israel) from day 1 and through marketing. The active ingredient in the feed additive is insulin derivatives (amino acid complex degraded ex-vivo from insulin with trypsin designated amino acid complex, AAC). The active ingredient is encapsulated within various coatings as described hereinbelow.

### Materials and Methods

The experiment was conducted in Kibbutz Gazit Experimental Facility located in Israel.

The experiment was conducted using either increasing or steady state dosing levels of AAC with two types of coating:
1. Thermal (T): Core of Sugar spheres, coated with AAC, coated with maltodextrin 18 and maltodextrin 6
2. Enteric (E): Core of Sugar spheres, coated with AAC, coated with Eudragit coated with maltodextrin 18 and maltodextrin 6

### Housing

Floor pen facilities were used during the trial. The house is closed with two layers of polythene and a shade net to control temperature fluctuations. Average chick density per pen in the house was 14 chicks per m². House was equipped with pan feeders, bell drinkers and built up pine sawdust. Feed and water were available to the birds *ad libitum*.

### Diets and Dietary Treatments

Ross 308 one day old chicks were placed in the trial facility and divided into 5 experimental groups with 6 replicates (62 birds per pen) All birds were fed a basal standard commercial diet from day 1 (following hatching) and through marketing at day 36. Diets were primarily composed of corn, wheat, soybean meal, vitamins, antibiotic growth promoter and coccidiostat premix. Pre-starter diets were given from day 1-7; starter diet till day 17; grower diet through day 27 and finisher feed till marketing at 36 days. Feed ingredients are summarized in Table 1.

**Table 1: Feed composition**

| **Calculated Analysis** | **Pre-Starter** | **Starter** | **Grower** | **Finisher** |
|---|---|---|---|---|
| Protein % | 22.5 | 20.0 | 20.0 | 18.5 |
| Ca% | 1.0 | 1.0 | 0.95 | 0.95 |
| Phosphorus % | 0.63 | 0.62 | 0.58 | 0.57 |
| Fat % | 6.04 | 6.48 | 7.35 | 8.12 |
| Fiber % | 3.49 | 3.27 | 3.27 | 3.15 |
| Ash % | 5.77 | 5.51 | 5.3 | 5.16 |
| Salt % | 0.49 | 0.49 | 0.45 | 0.38 |
| Energy (kcal/kg) | 3050 | 3120 | 3180 | 3250 |

### Dietary treatments included:

Group A: Decreasing dose accumulating to total of 3 IU per chick of insulin equivalent of AAC with thermal and enteric coating (T + E).
Group B: Decreasing dose accumulating to total of 3 IU per chick of insulin equivalent of AAC with thermal coating (T).
Group C: Steady state dose accumulating to total of 5 IU per chick of insulin equivalent of AAC with T + E coating;
Group D: Steady state dose accumulating to total of 5 IU per chick of insulin equivalent of AAC with T coating.
Control group: Basal diet.

### Measurements

Total weight of chicks and feed consumption per pen were measured at 7, 17, 27 and 35 days of age. Mortalities were collected daily and weighed. Feed conversion and intake were corrected in all periods by adjusting for mortality, weight and number. Feed conversion (FC) was determined until day 27. Technical problem in measuring feed weight distribution in pens from day 27 and through day 35 prevented result analysis for FC at day 35. Processing measurements were collected at day 36. Total of 40 birds were used for carcass parameter study. Birds were weighed prior to processing. Processing was manual and live body weight (BW), carcass weight, and cut up weights were measured and recorded.

### Statistical Analyses

Data was analyzed separately using the general linear models procedures of JMP® statistical discovery software (SAS Institute NC USA).

Due to the application of a control treatment, two separate analyses were performed to describe the dietary effects on broiler performance. A dose coating factorial analysis was run to explore main effects and their interactions. A second analysis was run that included all treatments. This analysis enabled comparisons between each treatment and control diet.

Differences between means were tested using *t* tests, and significance (defined by different letters) was p<0.05 unless otherwise stated (JMP, 2003).

### Results and Discussion

### Effect of AAC on body weight (BW) and feed conversion (FC)

At day 7, a significant effect of all treatments compared to the control group was determined for body weight gain, with group A demonstrating a statistical advantage. Average BW at day 17 and through marketing indicated similar significant improvement in BW of all treated groups as compared to control. However, no differences were found in FC between treatments (Tables 2 and 3).

**Table 2: Effect of treatments on BW (g)**

| Group/Day | 7 | 17 | 27 | 35 |
|---|---|---|---|---|
| A | 184.0±1.73 a | 607.8±5.9 a | 1352.2±9.3 a | 2189.5±16.7 a |
| B | 178.8±1.73 b | 594.5±5.9 a | 1320.8±9.3 b | 2136.0±16.7 bc |
| C | 179.5±1.60 ab | 599.9±5.5 a | 1334.4±8.6 ab | 2156.2±15.5 bc |
| D | 182.0±1.60 ab | 600.2±5.5 a | 1325.1±8.6 b | 2165.2±15.5 ab |
| Control | 171.4±1.73 c | 565.5±5.9 b | 1278.3±9.3 c | 2111.3±16.7 c |

**Table 3: Effect of treatment on FC (kg/kg)**

| Group//Day | 7 | 17 | 27 |
|---|---|---|---|
| A | 0.896 ± 0.01 | 1.27 ± 0.02 ab | 1.47 ± 0.02 |
| B | 0.888 ± 0.01 | 1.32 ± 0.02 b | 1.51 ± 0.02 |
| **C** | 0.882 ± 0.01 | 1.28 ± 0.02 ab | 1.45 ± 0.02 |
| D | 0.873 ± 0.01 | 1.29 ± 0.02 ab | 1.46 ± 0.02 |
| Control | 0.888 ± 0.01 | 1.23 ± 0.02 a | 1.45 ± 0.02 |

Factorial effects of BW at marketing (excluding control group) indicated an advantage to the decreased dose using thermal and enteric (T + E) coating (Tables 4-6). No effect was found in FC (Tables 7-9).

**Table 4: Factorial Effect on BW**

| Group/day | 7 | 17 | 27 | 35 |
|---|---|---|---|---|
| A | 178 ± 1.8 b | 594.5 ± 6.1 | 1320 ± 9.7 b | 2136.5 ± 17.9 b |
| B | 184.0 ± 1.8 a | 607 ± 6.1 | 1352 ± 9.7 a | 2189 ± 17.9 a |
| C | 182.0 ± 18 a | 600 ± 6.1 | 1325 ± 9.7b | 2165 ± 17.9 ab |
| D | 179.5 ± 1.6 b | 599 ± 6.1 | 1334 ± 9.7 ab | 2156 ± 16.6 ab |
| Pr>f | 0.07 | | 0.06 | |

**Table 5: Dose Effect on BW**

| Dose/Days | 7 | 17 | 27 | 35 |
|---|---|---|---|---|
| Decreased | 181.4 ± 1.2 | 601.2 ± 4.3 | 1336.5 ± 6.9 | 2162.8 ± 12.7 |
| Constant | 180.7 ± 1.2 | 600.0 ± 4.2 | 1329.7 ± 6.6 | 2160.7 ± 12.2 |

**Table 6: Coat Effect on BW**

| Coating/days | 7 | 17 | 27 | 35 |
|---|---|---|---|---|
| T | 180.4 ± 1.2 | 597.4 ± 4.3 | 1322.9 ± 6.9 b | 2150 ± 12.6 |
| T+E | 181.7 ± 1.2 | 604.8 ± 4.2 | 1343.3 ± 6.6 a | 2172 ± 12.2 |

**Table 7: Factorial Effect on FC**

| Group/day | 7 | 17 | 27 |
|---|---|---|---|
| A | 0.88 ± 0.001 | 1.32 ± 0.03 | 1.51 ± 0.02 |
| B | 0.89 ± 0.001 | 1.28 ± 0.03 | 1.47 ± 0.02 |
| C | 0.87 ± 0.001 | 1.28 ± 0.03 | 1.46 ± 0.02 |
| D | 0.88 ± 0.001 | 1.27 ± 0.03 | 1.45 ± 0.02 |

**Table 8: Dose Effect on FC**

| Dose/days | 7 | 17 | 27 |
|---|---|---|---|
| Dec | 0.89 ± 0.06 | 1.30 ± 0.02 | 1.49 ± 0.017 |
| Constant | 0.88 ± 0.06 | 1.28 ± 0.02 | 1.46 ± 0.017 |

**Table 9: Coating Effect on FC**

| Coating | 7 | 17 | 27 |
|---|---|---|---|
| T | 0.88 ± 0.06 | 1.30 ± 0.02 | 1.48 ± 0.017 |
| T+E | 0.89 ± 0.06 | 1.28 ± 0.02 | 1.46 ± 0.017 |

### Effects of the AAC on Body Composition

Live weight of the control group and group A was significantly higher than the live weight of group D. Carcass weight was similar for all treatments; however, percentage of carcass weight was greater in group D as compared to control and group A. No significant differences were found between control and treated groups in breast yield. Thigh yield was greatest in the control, group A and group C as compared to group D (Table 10).

**Table 10: Effect of AAC on carcass yields at day 35**

| | Control | Group A | Group C | Group D |
|---|---|---|---|---|
| Live weight | 2215 ± 9.1 a | 2217 ± 9.1 a | 2196.8 ± 9.1 ab | 2183.8 ± 9.1 b |
| Carcass weight | 1583.5 ± 7.7 | 1589.8 ± 7.6 | 1585.4 ± 7.5 | 1588.9 ± 7.9 |
| Carcass (% live weight) | 71.5 ± 0.3 b | 71.7 ± 0.3 b | 72.2 ± 0.3 ab | 72.8 ± 0.3 a |
| | | | | |
| Breast yield (g) | 451.0 ± 4.1 | 455.3 ± 4.1 | 457.7 ± 4.3 | 448.6 ± 4.1 |
| Breast yield (%live weight) | 20.4 ± 0.17 | 20.5 ± 0.17 | 20.8 ± 0.18 | 20.5 ± 0.18 |
| Breast yield (% carcass) | 28.4 ± 0.2 | 28.6 ± 0.2 | 28.9 ± 0.2 | 28.2 ± 0.2 |
| | | | | |
| Thigh Yield (g) | 671.2 ± 4.6 a | 673.7 ± 4.5 a | 668.6 ± 4.7 ab | 657.0 ± 4.1 b |
| Thigh Yield (%lw) | 30.3 ± 0.2 | 30.4 ± 0.2 | 30.4 ± 0.2 | 30.1 ± 0.2 |
| Thigh Yield (%carcass) | 42.4 ± 0.2 (a) | 42.4 ± 0.2 (a) | 42.2 ± 0.2 a | 41.4 ± 0.2 b |
| | | | | |
| Legs (g) | 89.0 ± 0.8 a | 89.1 ± 0.8 a | 85.9 ± 0.8 b | 85.1 ± 0.8 b |
| Legs (% lw) | 4.0 ± 0.03 a | 4.0 ± 0.03 a | 3.9 ± 0.03 b | 3.9 ± 0.03 b |
| Legs (%carcass) | 5.6 ± 0.04 (a) | 5.6 ± 0.04 a) | 5.4 ± 0.05 b | 5.4 ± 0.05 b |

### Example 2: Effect of Insulin on Growth Performance of Young Broiler Chicks

The object of the following study was to determine the effect of insulin preparations on weight gain, feed efficiency, body composition and intestine development of broiler chicks, obtaining basal fed mash diet according to NRC-94 nutrition guide book recommendations.

### Experimental Procedure

### Diets

Commercial chicken starter diet produced by Brown & S., Ltd. feed mill and formulated to meet NRC-94 requirements for broiler chicks was used in the study. Feeding was ad libitum in mash form starting when the chicks were one day old. Table 11 summarizes the calculated analyses of the diet.

**Table 11: Calculated analyses of the broiler' Starter crumble diet**

| Metabolically available Energy, kcal/kg | 2980 |
|---|---|
| Crude Protein, % | 21.0 |
| Calcium, % | 0.95 |
| Phosphorus, % | 0.72 |
| Fat, % | 4.0 |
| Fiber, % | 3.5 |
| Sulfur Amino acids, % | 0.90 |
| Lysine, % | 1.18 |
| Arginine, % | 1.4 |

Insulin was provided as encapsulated preparation (Insumeal™, Nutrinia, Ltd., Israel) when indicated. The Insumeal™ additive components are:
1. Insulin Actrapid® HM (ge) - Biosynthetic Human Insulin, Manufacture by Novo Nordisk, HM
2. Maltodextrin - Polysaccharide
3. Vitamin C

The placebo consisted of Corn flour and Maltodextrin.

Addition of Insumeal™ to the broilers' diet was terminated when they were one week old. Afterwards, the chicks received regular broiler diet without insulin until the end of the study at 28 days after hatching.

### The experimental treatments were as follows:

1. Control, without any supplementation of insulin preparation.
2. Experimental treatment with supplementation of insulin preparation of 0.23 units per 14 kg of the diet. Total consumption per bird per study - 0.004 IU of insulin.
3. Experimental treatment with supplementation of insulin preparation of 1.14 units per 14 kg of the diet. Total consumption per bird per study - 0.02 IU of insulin.
4. Experimental treatment with supplementation of insulin preparation of 5.71 units per 14 kg of the diet. Total consumption per bird per study - 0.1 IU of insulin.
5. Experimental treatment with supplementation of insulin preparation of 28.57 units per 14 kg of the diet. Total consumption per bird per study - 0.5 IU of insulin.
6. Experimental treatment with supplementation of insulin preparation of 142.86 units per 14 kg of the diet. Total consumption per bird per study - 2.5 IU of insulin.

### Chicks

One day-old broiler chicks were distributed into the above described six treatments with 4 replicate groups for each treatment, 10 chicks in each group. The chicks were located in heated batteries in climate-regulated room with constant temperature of 22°C and 24 hours of light till age of 28 days (end of the trial).

Individual body weight and feed consumption on a group basis was recorded weekly. At the end of the trial, feed intake and feed efficiency were calculated.

At the age of 7 and 28 days 2 chicks with average body weight from each group were killed and breast muscle, abdominal fat and intestine was weighed individually and calculated as % of body weight. At the age of 7 days the samples of intestine were taken for histological investigation in a laboratory at the Faculty of Agricultural, Food and Environmental Quality Sciences, Rehovot Israel.

### Results

The growth performance of chicks at the age period of 1-28 days is shown in Table 12. Significant differences were found between control and experimental treatments in final body weight and weight gain at the entire growth periods till 28 days after hatching.

Feed intake was almost equal between treatments and feed efficiency (g feed/g weight gain) was significantly better for chicks fed with the insulin supplement.

It need to be point out that insulin preparation was supplemented to the chicks' diet only at the first 7 days of their life but the enhanced growth was measured till the end of the trial. Moreover, the differences between treated and non-treated chicks increased with age.

**Table 2: Growth performance of the 28-d old chicks raised on diets with different amounts of insulin preparation**

| Parameter | Insulin Treatment (IU) | | | | | |
|---|---|---|---|---|---|---|
| | Control | 0.004 | 0.02 | 0.1 | 0.5 | 2.5 |
| Body weight 7 days, g | 169 b | 174 ab | 180 a | 170 b | 172 b | 174 ab |
| Weight gain | 124 b | 129 ab | 135 a | 126 b | 127 b | 129 ab |
| Feed intake, g | 169 | 179 | 178 | 180 | 178 | 171 |
| Feed efficiency, g/g | 1.36 ab | 1.39 ab | 1.32 b | 1.43 a | 1.41 ab | 1.32 b |
| | | | | | | |
| Body weight 14 days, g | 496 c | 520 b | 538 a | 525 ab | 524 ab | 519 b |
| Weight gain | 451 c | 475 b | 493 a | 480 ab | 479 ab | 474 b |
| Feed intake, g | 602 | 619 | 626 | 621 | 629 | 615 |
| Feed efficiency, g/g | 1.34 a | 1.30 ab | 1.27 b | 1.29 ab | 1.31 ab | 1.30 ab |
| | | | | | | |
| Body weight 21 days, g | 983 b | 1025 a | 1044 a | 1028 a | 1032 a | 1016 a |
| Weight gain | 938 b | 980 a | 999 a | 983 a | 986 a | 971 ab |
| Feed intake, g | 1353 | 1368 | 1381 | 1369 | 1373 | 1339 |
| Feed efficiency, g/g | 1.44 a | 1.40 ab | 1.38 b | 1.39 ab | 1.39 ab | 1.38 b . |
| | | | | | | |
| Body weight 28 days, g | 1543 b | 1602 a | 1607 a | 1612 a | 1601 a | 1581 ab |
| Weight gain | 1498 b | 1558 a | 1563 a | 1568 a | 1557 a | 1536 ab |
| Feed intake, g | 2348 | 2332 | 2329 | 2334 | 2336 | 2290 |
| Feed efficiency, g/g | 1.57 a | 1.50 b | 1.49 b | 1.49 b | 1.50 b | 1.49 b |

Body composition of the 28-d old chicks is shown in Table 3. Significant difference in the size of breast muscle was found between control and experimental treatments with insulin preparation in their diets. The weight of treated chick intestine was also significantly higher compared to chicks that did not receive insulin in their diet.

**Table 3: Effect of different amounts of dietary insulin preparation on the size of chick's body parts at 28-d old (in % of live Body Weight).**

| Parameters | Insulin treatment (IU) | | | | | |
|---|---|---|---|---|---|---|
| | Control | 0.004 | 0.02 | 0.1 | 0.5 | 2.5 |
| Breast muscle | 1.61 c | 17.1 b | 17.9 ab | 17.4 ab | 18.1 a | 17.1 b |
| Intestine | 6.15 b | 7.19 a | 6.89 a | 6.71 ab | 6.72 ab | 6.84 a |
| Abdominal fat | 1.32 ab | 1.32 ab | 1.33 ab | 1.17 b | 1.34 ab | 1.38 a |

### Example 3: Insulin Encapsulated Preparation (Insumeal™)

Insumeal™, the insulin composition used in the example of the present invention is a dry powder composed of microencapsulated insulin, within a matrix of Maltodextrin (MD) and Vitamin C. The micro-encapsulation process enables insulin bioactivity protection until its consumption by the animal.

The product contains three components:

**Insulin:** Actrapid® HM (ge), biosynthetic Human Insulin, Solution for Injection, Concentration of 100 IU/ml, manufactured by Novo Nordisk.

**Maltodextrin 18 (MD)**: corn starch polysaccharide, manufacture by Cargill Ltd. **Ascorbic Acid - 1 %**

The production was prepared according to the following stages:

| **Stage 1** | | **Example** |
|---|---|---|
| The core: | MD 18 | 600 gram |
| The solution: | Insulin | 2000 IU |
| | NaCl | 833 ml |
| | MD 18 | 400 gram |

The product of stage 1 was further processed in stage 2:

| **Stage 2** | | **Example** |
|---|---|---|
| The core: | Stage 1 product | 800 gram |
| The solution: | MD 18 | 88.7 |
| | Water | 207.4 |

The product of stage 2 was further processed in stage 3:

| **Stage 3** | | **Example** |
|---|---|---|
| The core: | Stage 2 product | 800 gram |
| The solution: | MD 18 | 80 gram |
| | Water | 207.4 |
| | Vitamin C | 8.9 |

The insulin concentration in the final formulation is 1.62 IU per gram dry product.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

## Claims

1. Use of a feed supplement comprising insulin for at least one of improved cumulative weight gain and improved feed conversion efficiency of poultry, wherein the feed supplement is administered at a total cumulative dose of insulin of 1 IU or less per individual chick.

2. The use according to claim 1, for increasing the intestinal mass of the poultry compared to the intestinal mass of poultry not receiving the feed supplement.

3. The use according to claim 1, wherein the feed supplement is administered at a total cumulative dose of insulin of about 0.51U per chick or less.

4. The use according to claim 3, wherein the feed supplement is administered at a total cumulative dose of insulin of from about 0.004 IU to about 0.5 IU per chick.

## Patentansprüche

1. Verwendung eines Futterzusatzes umfassend Insulin für mindestens eines von verbesserter Gesamtgewichtzunahme und verbesserter Futterverwertungseffizienz bei Geflügel, wobei der Futterzusatz in einer Gesamtkumulationsdosis an Insulin von 1 IE oder weniger pro einzelnem Hühnchen verabreicht wird.

2. Verwendung nach Anspruch 1, zur Erhöhung der intestinalen Masse des Geflügels im Vergleich zu der intestinalen Masse von Geflügel, das den Futterzusatz nicht erhält.

3. Verwendung nach Anspruch 1, wobei der Futterzusatz in einer Gesamtkumulationsdosis an Insulin von ungefähr 0,5 IE oder weniger pro Hühnchen verabreicht wird.

4. Verwendung nach Anspruch 3, wobei der Futterzusatz in einer Gesamtkumulationsdosis an Insulin von ungefähr 0,004 IE bis ungefähr 0,5 IE pro Hühnchen verabreicht wird

## Revendications

1. Utilisation d'un complément alimentaire comprenant de l'insuline pour au moins de : gain de poids cumulé amélioré et efficacité améliorée de la conversion des aliments pour la volaille, dans laquelle le complément alimentaire est administré à une dose d'insuline cumulative totale de 1 UI ou moins par poussin individuel.

2. L'utilisation selon la revendication 1 pour augmenter la masse intestinale de la volaille par rapport à la masse intestinale de volaille ne recevant pas le complément alimentaire.

3. L'utilisation selon la revendication 1 dans laquelle le complément alimentaire est administré à une dose d'insuline cumulative totale d'environ 0.5 UI ou moins par poussin.

4. L'utilisation selon la revendication 3, dans laquelle le complément alimentaire est administré à une dose d'insuline totale d'environ 0.004 UI à environ 0.5 UI ou moins par poussin.
